# EUROPÄISCHE PATENTSCHRIFT

(11) **EP 4 337 146 B1**
(45) Veröffentlichungstag und Bekanntmachung des Hinweises auf die Patenterteilung: **01.10.2025**
(21) Anmeldenummer: 22727820.7
(22) Anmeldetag: 04.05.2022
(51) Int. Cl.: A61F 2/58, A61F 2/68

(54) **EIGENKRAFTHANDPROTHESE MIT ZEITVERZÖGERUNG**
BODY-POWERED HAND PROSTHESIS HAVING A TIME DELAY
PROTHÈSE DE MAIN ACTIVÉE PAR L'ÉNERGIE DU CORPS PRÉSENTANT UN RETARD TEMPOREL

(30) Priorität: 10.05.2021 CH 5242021
(43) Veröffentlichungstag der Anmeldung: 20.03.2024
(73) Patentinhaber: Appsocial.org Stiftung, 8032 Zürich (CH)
(72) Erfinder: TROJAN, Andreas, 8032 Zürich (CH)
(74) Vertreter: Keller Schneider Patent- und Markenanwälte AG
(86) Internationale Anmeldenummer: PCT/EP2022/061957
(87) Internationale Veröffentlichungsnummer: WO 2022/238193

(56) Entgegenhaltungen:
- AT-B- 293 605
- DE-A1- 2 426 787
- DE-C- 821 690

## Beschreibung

### Technisches Gebiet

Die vorliegende Erfindung beschreibt eine mechanische Eigenkrafthandprothese mit einer Aufzieh- und Greifeinrichtung, wobei die Aufzieh- und Greifeinrichtung ein Aufziehmittel und ein Greifmittel umfasst, sodass nach Betätigung des Aufziehmittels ein Gelenk in Richtung einer Handfläche mittels Greifmittel bewegbar ist, wobei auf elektromechanische Bauteile verzichtet wird.

### Stand der Technik

Heute sind bevorzugt Prothesen, umfassend einen Prothesenschaft mit einer Exoprothese, beispielsweise in Form einer Handprothese gefragt, welche fremdkraftbetätigt sind. Derartige Exoprothesen können mindestens eine Bewegung ausführen und somit mindestens eine Bewegungsfunktion liefern. Dabei wird meist eine Prothesensteuerungsvorrichtung mit der Exoprothese verbunden, mittels welcher mindestens ein Prothesenmotor angesteuert wird, sodass mindestens ein Gelenk an der Exoprothese bewegt werden kann. Im Falle einer Handprothese wird so die Bewegung mindestens eines Gelenkes, bevorzugt des Daumengelenkes, nachgebildet, womit eine Greifbewegung möglich ist.

Es sind einige Stand der Technik Dokumente mit Protheseneinrichtungen der oberen Extremität bekannt, welche unterschiedliche Komplexitätsgrade aufweisen, angefangen von einfachen Haken, über einfache Greifer verschiedener Formen zu motorisch angetriebenen, oft auch myoelektrisch gesteuerten Handprothesen.

Neben hochkomplexen, sogar computergesteuerten, sensorbasierten Handprothesen existiert weiterhin ein Bedarf an vergleichsweise einfachen, mechanischen Handprothesen ohne viele Funktionen. Diese werden heute an so genannten Kraftzugbandagen befestigt und durch diese betätigt. Derartige Prothesen werden Eigenkraftprothesen genannt. Derartige Eigenkraftprothesen verzichten auf den Einsatz von zusätzlichen antreibbaren Motoren, wie Elektromotoren.

Beispielsweise mittels einer Schulterbewegung kann eine Öffnung einer Greifvorrichtung durch das Spannen einer Feder erreicht werden. Nach dem Öffnen der Greifvorrichtung wird durch eine weitere Bewegung der Schulter ein Greifvorgang ausgelöst. Dieser bleibt solange bestehen, bis die Greifvorrichtung wieder geöffnet wird. Das Greifen und Loslassen muss vom Prothesenträger aktiv ausgelöst werden, wenn gewünscht.

Grundsätzlich sind verschiedene Eigenkraftprothesen in der DE102015116133, in der DE821690, Gestänge und Kurvenscheibe und DE2607499, selbstsperrendes Getriebe, beschrieben. Die Mechanik reagiert auf einen Auslöser und es sind in der Regel Motoren und/oder Sensoren nötig, mit entsprechenden Steuerungsvorrichtungen, womit ein Öffnen und Schliessen eines Gelenkes durchgeführt wird.

Die DE821690 offenbart eine mechanische Eigenkrafthandprothese mit einer Aufzieh- und Greifeinrichtung, wobei auf elektromechanische Bauteile verzichtet wird. Die DE2426787 offenbart eine Sicherheitsvorrichtung für eine künstliche Hand mit durch Motor und Getriebe beweglichen Fingern. Die AT293605 offenbart eine Einrichtung zum Öffnen der Greiffinger einer mechanischen Kunsthand bei Auftreten einer einen Grenzwert übersteigender Zugbeanspruchung.

Die Handprothese aus DE2426711 weist Seilzüge auf und nutzt einen Motor zum Zug der Seilzüge, um eine direkt an die Auslösung gekoppelte Spannbewegung zu erreichen. Ähnliches gilt für DE3045271, worin eine von Hand verstellbare Prothese einer Hand zum Erfassen von Gegenständen gezeigt ist.

In der DE102016014090 ist eine einfache mechanische Handprothese offenbart, welche eine Relativbewegung zweier Prothesenteile zueinander nach Überwindung einer Federkraft erlaubt, wobei die Stellung durch dauerhaftes Einrasten erreicht wird.

In der EP45818 ist eine Handprothese gezeigt, welche eine Mechanik aufweist, die eine zeitliche Einstellung des Wechsels von einer zur anderen Griffart erlaubt. Auch hier wird ein Motor zur Erzeugung unterschiedlicher Bewegungen eingesetzt, sodass weder die Griffbewegung noch die Auflösung des Griffes mechanisch abläuft.

Die bislang bekannten rein mechanischen Handprothesen zeigen keine zeitlich gesteuerte Bewegung. Dies ist bislang nur durch die zusätzliche Verwendung von Elektronik erreicht worden.

Es soll eine Handprothese geschaffen werden, welche einen rein mechanisch betriebenen Mechanismus umfasst. Ohne Batterien, Mikrocontroller und/oder Motoren, soll eine Greifbewegung für einen definierten Zeitraum von wenigen Sekunden nach dem Auslösen gehalten werden. Das Auslösen erfolgt rein mechanisch und wird auch mechanisch beendet.

### Darstellung der Erfindung

Die vorliegende Erfindung hat sich zur Aufgabe gestellt eine einfache rein mechanisch ausgestaltete und kostengünstige Eigenkrafthandprothese zu schaffen, welche ohne Steuerelektronik auskommt und ohne elektrotechnisches Wissen einfach herstellbar ist, wobei eine Rückstellbewegung ohne Eingriff eines Prothesenträgers zu gegebener Zeit erfolgt.

Eine mechanisch robuste und wenig störanfällige Eigenhandkrafthandprothese mit wenig Gewicht ist das Ziel, wobei bevorzugt eine zeitverzögerte Rückstellung des Daumengelenkes nach einer definierten Wartezeit erreicht ist, sodass das Daumengelenk automatisiert zurück in eine Offenstellung gebracht wird.

Eine solche Eigenkrafthandprothese ist vor allem für den Einsatz beim Sport, insbesondere bei Ballsportarten wie Tennis oder Badminton einsetzbar, da das Greifen eines Balles durch die zeitlich begrenzte Bewegung eines Daumengelenkes wichtig ist, beispielsweise beim Aufwurf für den Aufschlag oder die Angabe. Dafür muss der Ball nach einer gewissen Zeit automatisch durch die Eigenkrafthandprothese freigegeben werden, damit er geworfen werden kann.

Natürlich spielen hier auch Gewichtseinsparungen und die Einfachheit der Bedienung eine Rolle für den Gebrauch derartiger Eigenkrafthandprothesen.

Zur Lösung der Aufgabe werden verschiedene Auslöse- und Greifeinrichtungen vorgestellt, welche für das Auslösen einer Greifbewegung, das zeitlich definierte Halten der Greifbewegung und das Lösen der Greifbewegung nach der definierten Wartezeit sorgen. Die Auslöse- und Greifeinrichtung verzichtet auf komplizierte Elektrotechnik und ist fest in die Eigenkrafthandprothese eingebaut.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben.
- Figur 1: zeigt eine schematische Perspektivansicht einer Prothese, wobei eine mechanische Auslöse- und Greifeinrichtung in der Eigenkrafthandprothese angedeutet ist.
- Figur 2a: zeigte eine perspektivische Ansicht einer Eigenkrafthandprothese in einer Offenstellung des Daumengelenkes mit angedeuteter Auslöse- und Greifeinrichtung, während
- Figur 2b: eine perspektivische Ansicht der Eigenkraftprothese aus Figur 2a in einer Geschlossenstellung des Daumengelenkes zeigt.
- Figur 3a: zeigt eine Explosionsdarstellung eine rein mechanische Auslöse- und Greifeinrichtung in einer ersten Ausführungsform, welche in
- Figur 3b: in einer perspektivischen zusammengebauten Ansicht gezeigt ist.

### Beschreibung

Die gesamte Prothese A, ist hier aus einem Prothesenschaft B, einer Kupplung C und einer Eigenkrafthandprothese D ohne zusätzliche elektromechanische Motoren zusammengesetzt. Mittels der Kupplung C am Prothesenschaft B können verschiedene Eigenkrafthandprothesen D auswechselbar an- und abgekoppelt werden. In den Körper der Eigenkrafthandprothese D ist eine Aufzieh- und Greifeinrichtung E integriert. Diese Aufzieh- und Greifeinrichtung E umfasst Aufziehmittel E1 und Greifmittel E2. Die Aufziehmittel E1 und Greifmittel E2 sind mechanisch miteinander verbunden. Es wird auf elektrotechnische Hilfsmittel verzichtet. Zur Andeutung der Besonderheit dieser Eigenkrafthandprothese ist eine Eieruhr symbolisch eingefügt, welche eine zeitlich versetzte Änderung von einer Geschlossenstellung in eine Offenstellung eines Gelenkes E3 andeutet.

Durch Betätigung des Aufziehmittels E1, Bewegung von einer Ruhestellung in eine Aufzugstellung, wird zeitlich direkt ein Greifprozess durch anschliessende Betätigung des mindestens einen Gelenkes E3 durch die Greifmittel E2 von der Offenstellung in die Geschlossenstellung des Gelenkes E3 ausgeführt. Entsprechend kann mit der Eigenkrafthandprothese D und dem Gelenk E3 beispielsweise ein Ball eingeklemmt gehalten werden, wenn das Gelenk E3 in der Geschlossenstellung ist.

Es sind verschiedene Aufziehmittel E1 und Greifmittel E2 vorstellbar, wobei hier entscheidend ist, dass nach einem Aufziehprozess durch Betätigung des Aufziehmittels E1, der mechanisch ausgelenkte Zustand des Aufziehmittels E1, die Aufzugsstellung, und der ausgelenkte Zustand des Greifmittels E2, die Geschlossenstellung des Gelenkes E3 für einige Sekunden gehalten wird.

Erst nach Ablauf der einmalig durch geeignete Wahl der Aufziehmittel E1 definierten Wirkzeit, also die Geschlossenstellung des Gelenkes E3, kehrt das Aufziehmittel E1 wieder in seine Ruhestellung zurück und das Greifmittel E2 geht in seine Offenstellung zurück. Hier ist das Gelenk E3 als Daumengelenk ausgebildet, es könnte aber auch ein anderes Gelenk gewählt sein, welches ein Einklemmen zwischen Gelenk und Handfläche D1.

Die bislang bekannten rein mechanischen Handprothesen zeigen keine automatische zeitlich gesteuerte Rückstellbewegung des Gelenkes E3 nach einer definierten Wirkzeit.

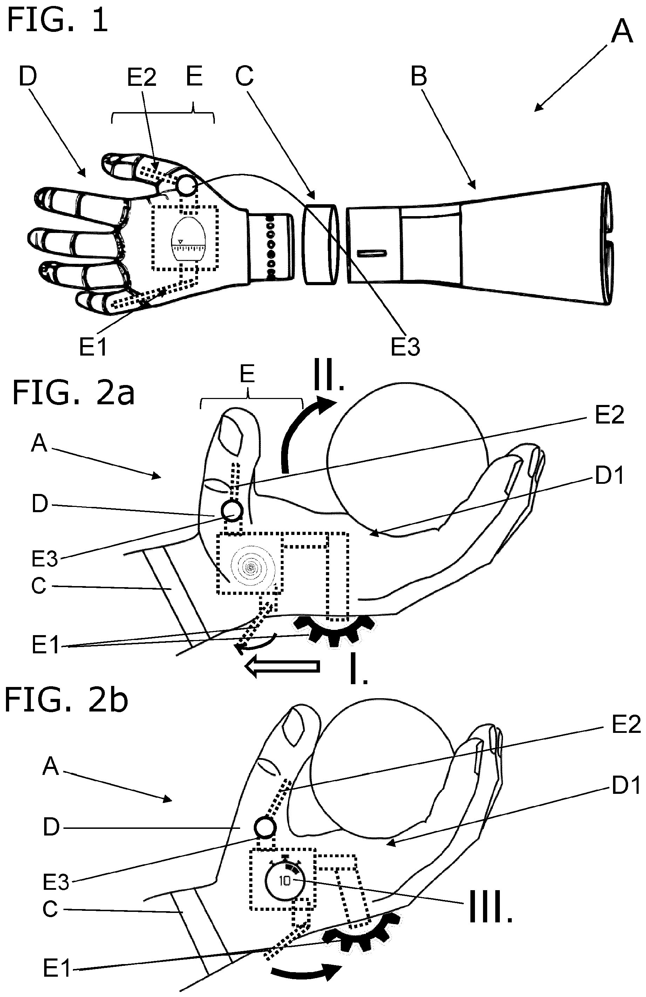

In Figur 2a ist die Offenstellung des Greifmittels E2 und des Gelenkes E3 gezeigt, wobei das Aufziehmittel E1 in seiner Ruhestellung ist. Ein Ball liegt auf der Handfläche D1 der Eigenkrafthandprothese D lose auf. Im ersten Schritt I, dem Aufziehvorgang I, werden vom Prothesenträger die Aufziehmittel E1 aufgezogen. Hier beispielhaft ist ein Aufziehhebel gezeigt, der bei Auslenkung einen Aufzugmechanismus betätigt. Eine weitere Möglichkeit ist die Ausbildung eines Aufziehrades als Aufziehmittel E1, welches ebenfalls einen Aufzugmechanismus antreibt. Die Bewegungsrichtung des Aufziehmittels E1 von seiner Ruhestellung in seine Aufzugsstellung ist mit einem geraden Pfeil angedeutet, wobei eine Auslenkung auch in eine andere Richtung erfolgen kann.

Der Aufzugmechanismus umfasst Rückstellmittel bzw. Rastmittel, sodass das Aufziehmittel E1 nach dem Aufziehen nach einer definierten Zeit wieder in seine Ruhestellung übergeht. Für die Aufziehmittel E1 bzw. den Rückstellmechanismus E11 gibt es verschiedene Ausführungsmöglichkeiten.

Mit dem Aufziehen des Aufziehmittels E1 wird der Greifvorgang II eingeleitet, wobei das Greifmittel E2 das Gelenk E3 in seine Geschlossenstellung auslenkt, was mit dem gebogenen Pfeil angedeutet ist. Der Ball wird direkt oder indirekt vom Greifmittel E2 auf der Handfläche D1 gegen mindestens einen Finger eingeklemmt.

Als Greifmittel E2 können mehrere Mechanismen gewählt werden, die dem Fachmann, ohne erfinderisch tätig zu werden, bekannt sind. Beispielsweise kann das Gelenk E3 durch mindestens ein Zugseil in die Geschlossenstellung ausgelenkt werden, wobei das Zugseil gegen eine Rückstellfeder gezogen wird, was hier nicht dargestellt ist. Vor allem sind Greifmittel E2 vorgesehen, die möglichst einfach aufgebaut sind und bevorzugt mittels Kunststoffspritzguss oder 3D-Druck hergestellt sind. Die Greifmittel E2 sind in den Körper der Eigenkrafthandprothese D integriert. Dadurch sind sie mechanisch geschützt unverlierbar gehalten.

Das Greifmittel E2 umfasst wenigstens einen Steg, der aus ästhetischen Gründen in einen Finger, hier in den Daumen integriert ist. Damit kann der gesamte Daumenkörper gegen den Ball und damit der Ball in Richtung Handfläche D1 gepresst werden, um den Ball zu halten.

Das Greifmittel E2 ist für eine definierte Zeit, eine Wartezeit III, von bevorzugt maximal zehn Sekunden, in der Geschlossenstellung des Gelenkes E3. Das Aufziehmittel E1 kann während dieser Wartezeit III in Aufzugstellung sein oder sich bereits in Ruhestellung zurückbewegen. Diese Zurückbewegung ist mit dem dicken Pfeil in Figur 2b angedeutet.

Nach Ablauf der Wartezeit III ist das Aufziehmittel E1 wieder zurück in seine Ruhestellung bewegt und das Greifmittel E2 in seiner Offenstellung, was dem Zustand von Figur 2a entspricht.

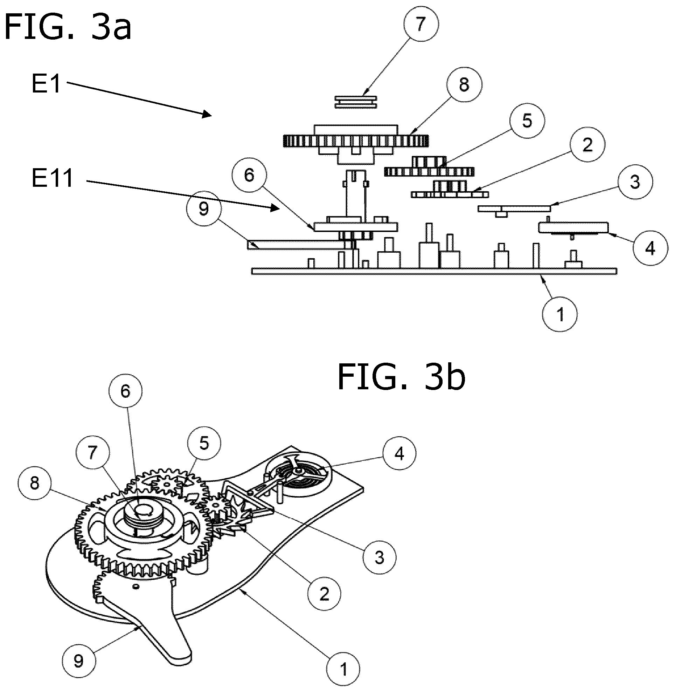

In Figur 3a und 3b ist eine Variante eines Aufziehmittels E1 zur Integration in die Eigenkrafthandprothese D gezeigt. Diese Idee stammt aus dem Bereich der Uhrmacherei, wo sogenannte Eieruhren ebenfalls die Einstellung einer Wartezeit erlauben. Auf einer Halterung 1, sind ein Ankerrad 2, ein Anker, eine Unruh mit Haarfeder 4, ein Zwischenrad 5, ein Aufzug 6, eine Kabeltrommel 7, ein Federhaus 8 und der Aufziehhebel 9 angeordnet. Wird der Aufziehhebel 9 in eine Aufzugsstellung ausgelenkt, wird von der Unruh 4 gesteuert eine Wartezeit ablaufen gelassen, bevor der Aufziehhebel 9 wieder zurück in seiner Ruhestellung ist.

Die Aufziehmittel E1 können aber auch andere Arten von Rückstellmechanismen E11 aufweisen, beispielsweise eine einfache Rückstellfeder in Form einer Spiralfeder oder einen Schneckenantrieb mit Rückstellfeder. Entscheidend ist, dass nach Betätigung des Aufziehhebels 9 oder des Aufziehrades eine zeitliche Verzögerung bis zur Rückführung in die Ruhestellung erreicht ist.

Dies kann auch durch Spannen mindestens eines Gummibandes, welches dann einrastet oder durch Eindrücken eines Formgedächtnispolymers erreicht werden. Ein derartiges Formgedächtnispolymer mit Einweg-Memory-Effekt, kann durch Eindrücken des Aufziehhebels 9 oder Betätigung des Aufziehrades durch einen geeigneten Aufbau eingedrückt werden. Dies kann mit einem Stempel oder Kolben geschehen, wobei eine Rückbewegung aufgrund des Einweg-Memory-Effekts je nach Wahl und Dicke des Formgedächtnispolymers zeitverzögert erfolgt.

Weiter sind Aufziehmittel E1 mit Rückstellmechanismen E11 einsetzbar, welche auf Pneumatikzylindern mit oder ohne integrierter Feder oder Gasdruckfedern basieren, wobei bei Aufziehvorgang I entsprechend durch den Aufziehhebel 9 oder das Aufziehrad ausgelenkt wird. Technisch aufwändiger aber machbar ist die Verwendung eines Hydraulikzylinders mit einem Fluid, wobei auch hier auf elektromechanische Aufbauten verzichtet wird und die rein mechanisch wirkende Aufzieh- und Greifeinrichtung E resultiert. Die Auslöse- und Greifeinrichtung E verzichtet auf komplizierte Elektrotechnik/Elektromechanik und ist fest in die Eigenkrafthandprothese D eingebaut.

Die Aufziehmittel E1 und die Greifmittel E2 sind gekoppelt, sodass die Greifmittel E2 nach dem Aufziehen der Aufziehmittel E1 betätigt werden und die Aufziehmittel E1 nach Ablauf der Wartezeit wieder in ihre Ruhestellung zurück gehen können.

### Bezugszeichenliste

A Prothese
B Prothesenschaft
C Kupplung für Exoprothese
D Eigenkrafthandprothese
   D1 Handfläche
E Aufzieh- und Greifeinrichtung
   E1 Aufziehmittel
      E11 Rückstellmechanismus
      1 Halterung
      2 Ankerrad
      3 Anker
      4 Unruh mit Haarfeder
      5 Zwischenrad
      6 Aufzug
      7 Kabeltrommel
      8 Federhaus
      9 Aufziehhebel
   E2 Greifmittel
   E3 Gelenk (insbesondere Daumengelenk)
I Aufziehvorgang
II Greifvorgang
III Wartezeit

## Patentansprüche

1. Mechanische Eigenkrafthandprothese (D) mit einer Aufzieh- und Greifeinrichtung (E), wobei die Aufzieh- und Greifeinrichtung (E) ein Aufziehmittel (E1) und ein Greifmittel (E2) umfasst, sodass nach Betätigung des Aufziehmittels (E1) ein Gelenk (E3) in Richtung einer Handfläche (D1) mittels Greifmittel (E2) bewegbar ist, wobei auf elektromechanische Bauteile verzichtet wird,
wobei
die Aufziehmittel (E1) und Greifmittel (E2) rein mechanisch wirkend miteinander verbunden ausgebildet in den Körper der Eigenkrafthandprothese (D) integriert sind und das Aufziehmittel (E1) einen Rückstellmechanismus (E11) derart umfasst,
dass durch Betätigung eines Aufziehhebels (9) oder eines Aufziehrades eine Auslenkung des Aufziehmittels von einer Ruhestellung in eine Aufzugsstellung dabei eine Auslenkung des Greifmittels (E2) und damit eines Gelenkes (E3) in eine Geschlossenstellung zur Durchführung eines Greifprozesses erreichbar ist,
**dadurch gekennzeichnet, dass**
das Greifmittel (E2) derart ausgestaltet ist, dass es für eine definierte Wartezeit (III) in der Aufzugsstellung verbleibt und das Aufziehmittel (E1) derart ausgestaltet ist, dass es die Rückstellung des Greifmittels (E2) erst nach Ablauf der Wartezeit (III) in eine Offenstellung erlaubt, womit ein zeitlich definiert gehaltener Greifvorgang ermöglicht ist, welcher automatisch ohne Eingreifen des Prothesenträgers nach der Wartezeit (III) gelöst wird, indem eine zeitlich gesteuerte Rückstellbewegung des Gelenkes (E3) und des Aufziehmittels (E1) erfolgt.

2. Mechanische Eigenkrafthandprothese (D) mit einer Aufzieh- und Greifeinrichtung (E) nach Anspruch 1, wobei das Aufziehmittel (E1) durch einen Prothesenträger aufziehbar ist und einen Aufziehhebel (9) oder ein Aufziehrad umfasst.

3. Mechanische Eigenkrafthandprothese (D) mit einer Aufzieh- und Greifeinrichtung (E) nach einem der vorhergehenden Ansprüche, wobei das Greifmittel (E2) derart gewählt ist, dass es eine Wartezeit (III) von höchstens zehn Sekunden ermöglicht.

4. Mechanische Eigenkrafthandprothese (D) mit einer Aufzieh- und Greifeinrichtung (E) nach einem der vorhergehenden Ansprüche, wobei das Aufziehmittel (E1) einen Rückstellmechanismus (E11) umfasst, insbesondere in Form einer Rückstellfeder oder einer Unruh mit Haarfeder (4).

5. Mechanische Eigenkrafthandprothese (D) mit einer Aufzieh- und Greifeinrichtung (E) nach einem der vorhergehenden Ansprüche, wobei das Aufziehmittel (E1) einen Rückstellmechanismus (E11) umfasst, insbesondere in Form eines Schneckenantriebs mit Rückstellfeder.

6. Mechanische Eigenkrafthandprothese (D) mit einer Aufzieh- und Greifeinrichtung (E) nach einem der vorhergehenden Ansprüche, wobei das Aufziehmittel (E1) einen Rückstellmechanismus (E11) umfasst, insbesondere mindestens ein Gummiband oder einen Körper eines Formgedächtnispolymers mit Einweg-Memory-Effekt.

7. Mechanische Eigenkrafthandprothese (D) mit einer Aufzieh- und Greifeinrichtung (E) nach einem der vorhergehenden Ansprüche, wobei das Aufziehmittel (E1) einen Rückstellmechanismus (E11) umfasst, insbesondere umfassend einen Pneumatikzylinder mit oder ohne integrierte Feder oder der Rückstellmechanismus (E11) auf einer Gasdruckfeder basiert.

8. Mechanische Eigenkrafthandprothese (D) mit einer Aufzieh- und Greifeinrichtung (E) nach einem der vorhergehenden Ansprüche, wobei das Greifmittel (E2) mindestens ein Zugseil umfasst, welches gegen eine Rückstellfeder gezogen wird, wobei das Gelenk (E3) auslenkbar ist.

9. Mechanische Eigenkrafthandprothese (D) mit einer Aufzieh- und Greifeinrichtung (E) nach einem der vorhergehenden Ansprüche, wobei das Greifmittel (E2) und das Aufziehmittel (E1) mittels Kunststoffspritzguss oder 3D-Druck aus Kunststoff hergestellt sind und in den Körper der Eigenkrafthandprothese (D) integriert sind.

10. Mechanische Eigenkrafthandprothese (D) mit einer Aufzieh- und Greifeinrichtung (E) nach einem der vorhergehenden Ansprüche, wobei das Greifmittel (E2) wenigstens einen Steg umfasst, der bevorzugt in einen Finger der Eigenkrafthandprothese (D) integriert ist, wodurch ein Gegenstand in Richtung Handfläche (D1) pressbar ist.

## Claims

1. Mechanical self-powered hand prosthesis (D) with a pulling and gripping device (E), wherein the pulling and gripping device (E) comprises a pulling means (E1) and a gripping means (E2) such that, after actuation of the pulling means (E1), a joint (E3) is movable in the direction of a palm (D1) by means of the gripping means (E2), wherein no electromechanical components are used, wherein the pulling means (E1) and gripping means (E2) are connected to each other in a purely mechanical manner and are integrated into the body of the self-powered hand prosthesis (D), and the pulling means (E1) comprises a return mechanism (E11) such that, by actuating a pulling lever (9) or a pulling wheel, the pulling means can be deflected from a rest position into a pulling position and, in doing so, a deflection of the gripping means (E2) and thus of a joint (E3) is obtainable into a closed position for carrying out a gripping process,
**characterised in that**
the gripping means (E2) is designed such that it remains in the pulling position for a defined waiting time (III) and the pulling means (E1) is designed such that it only allows the return of the gripping means (E2) into an open position after the waiting time (III) has elapsed, thereby enabling a time-defined held gripping process, which is automatically released after the waiting time (III) without intervention by the prosthesis wearer by means of a time-controlled return movement of the joint (E3) and the pulling means (E1).

2. Mechanical self-powered hand prosthesis (D) with a pulling and gripping device (E) according to claim 1, wherein the pulling means (E1) can be pulled by a prosthesis wearer and comprises a pulling lever (9) or a pulling wheel.

3. Mechanical self-powered hand prosthesis (D) with a pulling and gripping device (E) according to one of the preceding claims, wherein the gripping means (E2) is selected such that it allows a waiting time (III) of at most ten seconds.

4. Mechanical self-powered hand prosthesis (D) with a pulling and gripping device (E) according to one of the preceding claims, wherein the pulling means (E1) comprises a return mechanism (E11), in particular in the form of a return spring or a balance wheel with a hairspring (4).

5. Mechanical self-powered hand prosthesis (D) with a pulling and gripping device (E) according to one of the preceding claims, wherein the pulling means (E1) comprises a return mechanism (E11), in particular in the form of a worm drive with a return spring.

6. Mechanical self-powered hand prosthesis (D) with a pulling and gripping device (E) according to one of the preceding claims, wherein the pulling means (E1) comprises a return mechanism (E11), in particular at least one rubber band or a body made of a shape memory polymer with a one-way memory effect.

7. Mechanical self-powered hand prosthesis (D) with a pulling and gripping device (E) according to one of the preceding claims, wherein the pulling means (E1) comprises a return mechanism (E11), in particular comprising a pneumatic cylinder with or without an integrated spring, or the return mechanism (E11) is based on a gas pressure spring.

8. Mechanical self-powered hand prosthesis (D) with a pulling and gripping device (E) according to one of the preceding claims, wherein the gripping means (E2) comprises at least one pulling rope which is pulled against a return spring, wherein the joint (E3) is deflectable.

9. Mechanical self-powered hand prosthesis (D) with a pulling and gripping device (E) according to one of the preceding claims, wherein the gripping means (E2) and the pulling means (E1) are made of plastic by plastic injection moulding or 3D printing and are integrated into the body of the self-powered hand prosthesis (D).

10. Mechanical self-powered hand prosthesis (D) with a pulling and gripping device (E) according to one of the preceding claims, wherein the gripping means (E2) comprises at least one web, which is preferably integrated into a finger of the self-powered hand prosthesis (D), whereby an object can be pressed towards the palm (D1).

## Revendications

1. Prothèse de main mécanique activée par l'énergie du corps (D) avec un dispositif de levage et de préhension (E) sachant que le dispositif de levage et de préhension (E) comprend un moyen de levage (E1) et un moyen de préhension (E2) de telle manière qu'une fois le moyen de levage (E1) actionné, une articulation (E3) peut être déplacée en direction d'une paume (D1) au moyen du moyen de préhension (E2), sachant que l'on a renoncé à des composants électromécaniques,
sachant que le moyen de levage (E1) et le moyen de préhension (E2) constitués reliés entre eux agissant de façon purement mécanique sont intégrés dans le corps de la prothèse de main activée par l'énergie du corps (D) et le moyen de levage (E1) comprend un mécanisme de rappel (E11) de telle sorte qu'en actionnant un levier de levage (9) ou une roue de levage, on peut obtenir une déviation du moyen de levage d'une position neutre à une position de levage et de ce fait une déviation du moyen de préhension (E2) et avec cela d'une articulation (E3) dans une position Fermée par exécution d'un processus de préhension,
**caractérisée en ce que**
le moyen de préhension (E2) est constitué de telle manière qu'il reste pour un temps de retard défini (III) dans la position de levage et le moyen de levage (E1) est constitué de telle manière qu'il ne permet le rappel du moyen de préhension (E2) qu'après expiration du temps de retard (III) dans une position Ouverte, une opération de préhension maintenue dans un temps défini étant ainsi possible, laquelle est déclenchée automatiquement sans intervention du support de prothèse après le temps de retard (III), un mouvement de rappel géré dans le temps de l'articulation (E3) et du moyen de levage (E1) ayant lieu de ce fait.

2. Prothèse de main mécanique activée par l'énergie du corps (D) avec un dispositif de levage et de préhension (E) selon la revendication 1, sachant que le moyen de levage (E1) peut être ouvert par un support de prothèse et comprend un levier de levage (9) ou une roue de levage.

3. Prothèse de main mécanique activée par l'énergie du corps (D) avec un dispositif de levage et de préhension (E) selon l'une quelconque des revendications précédentes, sachant que le moyen de préhension (E2) est choisi de telle manière qu'il permet un temps de retard (III) de dix secondes au maximum.

4. Prothèse de main mécanique activée par l'énergie du corps (D) avec un dispositif de levage et de préhension (E) selon l'une quelconque des revendications précédentes, sachant que le moyen de levage (E1) comprend un mécanisme de rappel (E11), en particulier sous la forme d'un ressort de rappel ou d'un balancier avec ressort capillaire (4).

5. Prothèse de main mécanique activée par l'énergie du corps (D) avec un dispositif de levage et de préhension (E) selon l'une quelconque des revendications précédentes, sachant que le moyen de levage (E1) comprend un mécanisme de rappel (E11), en particulier sous la forme d'un système d'entraînement à vis sans fin avec ressort de rappel.

6. Prothèse de main mécanique activée par l'énergie du corps (D) avec un dispositif de levage et de préhension (E) selon l'une quelconque des revendications précédentes, sachant que le moyen de levage (E1) comprend un mécanisme de rappel (E11), en particulier au moins une bande de caoutchouc ou un corps d'un polymère à mémoire de forme avec un effet de mémoire unidirectionnel.

7. Prothèse de main mécanique activée par l'énergie du corps (D) avec un dispositif de levage et de préhension (E) selon l'une quelconque des revendications précédentes, sachant que le moyen de levage (E1) comprend un mécanisme de rappel (E11), comprenant en particulier un vérin pneumatique avec ou sans ressort intégré ou le mécanisme de rappel (E11) est basé sur un ressort de pression à gaz.

8. Prothèse de main mécanique activée par l'énergie du corps (D) avec un dispositif de levage et de préhension (E) selon l'une quelconque des revendications précédentes, sachant que le moyen de préhension (E2) comprend au moins un câble de traction, lequel est tiré contre un ressort de rappel, sachant que l'articulation (E3) peut être orientable.

9. Prothèse de main mécanique activée par l'énergie du corps (D) avec un dispositif de levage et de préhension (E) selon l'une quelconque des revendications précédentes, sachant que le moyen de préhension (E2) et le moyen de levage (E1) sont fabriqués en matière plastique moulée par injection ou en par moulage sous pression tridimensionnel et sont intégrés dans le corps de la prothèse de main activée par l'énergie du corps (D).

10. Prothèse de main mécanique activée par l'énergie du corps (D) avec un dispositif de levage et de préhension (E) selon l'une quelconque des revendications précédentes, sachant que le moyen de préhension (E2) comprend au moins une nervure, qui est intégrée de préférence dans un doigt de la prothèse de main activée par l'énergie du corps (D), un objet pouvant être pressé de ce fait en direction de la paume (D1).
